# EUROPÄISCHE PATENTSCHRIFT

(11) **EP 0 937 050 B1**
(45) Veröffentlichungstag und Bekanntmachung des Hinweises auf die Patenterteilung: **08.08.2007**
(21) Anmeldenummer: 97913182.8
(22) Anmeldetag: 29.10.1997
(51) Int. Cl.: C07D 239/52, C07D 239/56, A01N 43/54

(54) **HALOGENPYRIMIDINYLARYL(THIO)ETHER ALS PESTIZIDE**
HALOGEN PYRIMIDINYL ARYL (THIO)ETHERS AS PESTICIDES
HALOGENOPYRIMIDINYLARYL(THIO)ETHERS UTILISES COMME PESTICIDES

(30) Priorität: 11.11.1996 DE 19646407
(43) Veröffentlichungstag der Anmeldung: 25.08.1999
(73) Patentinhaber: Bayer CropScience AG, 40789 Monheim (DE)
(72) Erfinder: GAYER, Herbert, D-40789 Monheim (DE); GERDES, Peter, D-52080 Aachen (DE); HEINEMANN, Ulrich, D-42799 Leichlingen (DE); KRÜGER, Bernd-Wieland, D-51467 Bergisch Gladbach (DE); TIEMANN, Ralf, D-51375 Leverkusen (DE); DUTZMANN, Stefan, D-40764 Langenfeld (DE); HÄNSSLER, Gerd, D-51381 Leverkusen (DE); STENZEL, Klaus, D-40595 Düsseldorf (DE)
(86) Internationale Anmeldenummer: PCT/EP1997/005954
(87) Internationale Veröffentlichungsnummer: WO 1998/021189

(56) Entgegenhaltungen:
- EP-A- 0 398 692
- EP-A- 0 647 631
- DE-A- 4 443 641
- GB-A- 2 253 624

## Beschreibung

Die Erfindung betrifft neue Halogenpyrimidine, zwei Verfahren zu ihrer Herstellung und ihre Verwendung als Schädlingsbekämpfungsmittel.

Bestimmte Pyrimidine mit ähnlichem Substitutionsmuster, sowie deren fungizide Wirkung sind bereits bekannt geworden (GB-A 2253624). Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Halogenpyrimidine der allgemeinen Formel (I) gefunden, 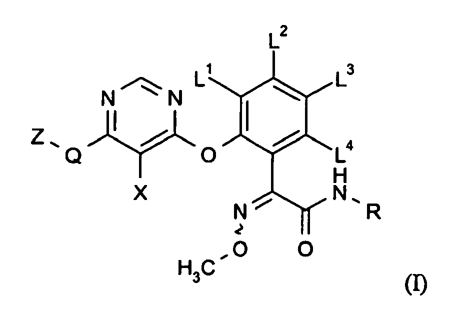 in welcher
- Z: für jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 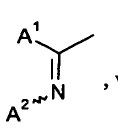 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
- R: für Wasserstoff oder Methyl steht,
- Q: für Sauerstoff oder Schwefel steht,
- X: für Fluor, Chlor, Brom oder Iod steht und
- L¹, L², L³ und L⁴: gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt.

Aryl steht für aromatische, mono oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Weiterhin wurde gefunden, dass man die neuen Halogenpyrimidine der allgemeinen Formel (I) erhält, wenn man
a) 2-(2-Hydroxy-phenyl)-2-methoxyimino-acetamide der Formel (II), 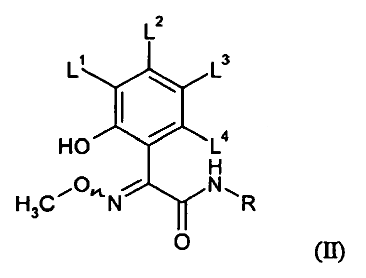 in welcher
   - R, L¹, L², L³ und L⁴: die oben angegebenen Bedeutungen haben,
   mit einem substituierten Halogenpyrimidin der allgemeinen Formel (III), 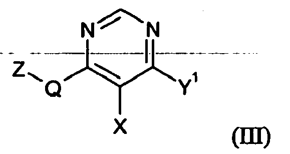 in welcher
   - Z, Q und X: die oben angegebenen Bedeutungen haben und
   - Y¹: für Halogen steht,
   gegebenenfalls in Gegenwart eines Verdünnungsmittels, gegebenenfalls in Gegenwart eines Säureakzeptors und gegebenenfalls in Gegenwart eines Katalysators, umsetzt, oder wenn man
b) Phenoxypyrimidine der allgemeinen Formel (IV) 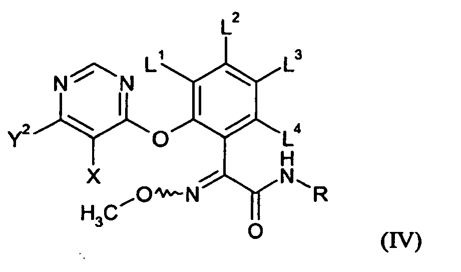 in welcher
   - R, X, L¹, L², L³ und L⁴: die oben angegebenen Bedeutungen haben und
   - Y²: für Halogen steht,
   mit einer Ringverbindung der allgemeinen Formel (V),

   Z-Q-H (V)

   in welcher
   - Z und Q: die oben angegebenen Bedeutungen haben,
   gegebenenfalls in Gegenwart eines Verdünnungsmittels, gegebenenfalls in Gegenwart eines Säureakzeptors und gegebenenfalls in Gegenwart eines Katalysators, umsetzt.

Schließlich wurde gefunden, dass die neuen Halogenpyrimidine der allgemeinen Formel (I) eine sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Gegenstand der Erfindung sind vorzugsweise Verbindungen der Formel (I), in welcher
- Z: für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl oder Ethyl substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl.#
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 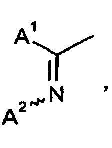 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
- R: für Wasserstoff oder insbesondere Methyl steht,
- Q: für Sauerstoff oder Schwefel steht,
- X: für Fluor oder Chlor steht und
- L¹, L², L³ und L⁴: gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio,
Difluorchlormethylthio, Trifluormethylthio, Trifluormethylsulfinyl oder Trifluormethylsulfonyl stehen.

In einer ganz besonders bevorzugten Gruppe von Verbindungen steht Z für gegebenenfalls substituiertes Phenyl.

In einer weiteren ganz besonders bevorzugten Gruppe von Verbindungen stehen
- L¹ und L³: unabhängig voneinander für Methyl und insbesondere Wasserstoff und
- L² und L⁴: für Wasserstoff.

Insbesondere sind Verbindungen der Formel (I) bevorzugt in denen X für Fluor steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Diese Restedefinitionen können untereinander, also auch zwischen den angegebenen Bereichen bevorzugter Verbindungen, beliebig kombiniert werden.

Die zur Durchführung des erfindungsgemäßen Verfahrens a) als Ausgangsstoffe benötigten 2-(2-Hydroxy-phenyl)-2-methoxyimino-acetamide sind durch die Formel (II) allgemein definiert. In dieser Formel (II) haben R, L¹, L², L³ und L⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R, L¹, L², L³ und L⁴ angegeben wurden.

Die Ausgangsstoffe der Formel (II) sind bekannt und können nach bekannten Verfahren hergestellt werden (vergleiche z.B. WO-A 9524396).

Die zur Durchführung des erfindungsgemäßen Verfahrens a) weiterhin als Ausgangsstoffe benötigten Halogenpyrimidine sind durch die Formel (III) allgemein definiert. In dieser Formel (III) haben Z, Q und X vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Z, Q und X angegeben wurden. Y¹ steht für Halogen, vorzugsweise für Fluor oder Chlor.

Die Ausgangsstoffe der Formel (III) sind bekannt-und/oder-können-nach-bekannten Methoden hergestellt werden (vergleiche z. B. DE-A 4340181; Chem.Ber., 90 <1957> 942, 951).

Die zur Durchführung des erfindungsgemäßen Verfahrens b) als Ausgangsstoffe benötigten Phenoxypyrimidine sind durch die Formel (IV) allgemein definiert. In dieser Formel (IV) haben R, X, L¹, L², L³ und L⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R, X, L¹, L², L³ und L⁴ angegeben wurden. Y² steht für Halogen, vorzugsweise für Fluor oder Chlor.

Die Ausgangsstoffe der Formel (IV) sind neu und ebenfalls Gegenstand der vorliegenden Anmeldung.

Die Phenoxypyrimidine der allgemeinen Formel (IV) werden erhalten (Verfahren b-1), wenn man 2-(2-Hydroxy-phenyl)-2-methoxyimino-acetamide der Formel (II) mit einem Trihalogenpyrimidin der allgemeinen Formel (VI) 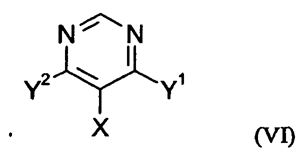 in welcher
- X, Y¹ und Y²: gleich oder verschieden sind und jeweils für Halogen stehen,
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens b-1) als Ausgangsstoffe benötigten Hydroxyverbindungen der Formel (II) sind bereits im Zusammenhang mit der Beschreibung des erfindungsgemäßen Verfahrens a) beschrieben worden.

Die weiterhin zur Durchführung des erfindungsgemäßen Verfahrens b-1) als Ausgangsstoffe benötigten Trihalogenpyrimidine sind durch die Formel (VI) allgemein definiert. In dieser Formel (VI) stehen X, Y¹ und Y² für Halogen, vorzugsweise für Fluor oder Chlor.

Die Trihalogenpyrimidine sind bekannt und/oder können nach bekannten Methoden hergestellt werden (vergleiche z. B. Chesterfield et al., J. Chem. Soc., 1955; 3478, 3480).

Die weiterhin zur Durchführung des erfindungsgemäßen Verfahrens b) als Ausgangsstoffe benötigten Ringverbindungen sind durch die Formel (V) allgemein definiert. In dieser Formel (V) haben Z und Q vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Z und Q angegeben wurden.

Die Ringverbindungen der Formel (V) sind bekannte Synthesechemikalien oder können nach einfachen Methoden hergestellt werden.

Als Verdünnungsmittel zur Durchführung der erfindungsgemäßen Verfahren a), b) und b-1) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Die erfindungsgemäßen Verfahren a), b) und b-1) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -alkoholate, -carbonate oder -hydrogencarbonate, wie beispielsweise Natriumhydrid, Natriumamid, Kalium-tert.-butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren a), b) und b-1) eignen sich alle Kupfer(I)-Salze, wie beispielsweise Kupfer(I)-chlorid, Kupfer(I)-bromid oder Kupfer(I)-iodid.

Die Reaktionstemperaturen können bei der Durchführung der erfindungsgemäßen Verfahren a), b) und b-1) in einem größeren Bereich variiert werden. Im Allgemeinen arbeitet man bei Temperaturen von -20°C bis 100°C, vorzugsweise bei Temperaturen von -10°C bis 80°C.

Zur Durchführung des erfindungsgemäßen Verfahrens a) zur Herstellung der Verbindungen der Formel (I) setzt man pro Mol des 2-(2-Hydroxy-phenyl)-2-methoxyimino-acetamides der Formel (II) im Allgemeinen 0,5 bis 15 Mol, vorzugsweise 0,8 bis 8 Mol substituiertes Halogenpyrimidin der Formel (III) ein.

Zur Durchführung des erfindungsgemäßen Verfahrens b) zur Herstellung der Verbindungen der Formel (I) setzt man pro Mol des Phenoxypyrimidins der Formel (IV) im Allgemeinen 0,5 bis 15 Mol, vorzugsweise 0,8 bis 8 Mol einer Ringverbindung der allgemeinen Formel (V) ein.

Zur Durchführung des erfindungsgemäßen Verfahrens b-1) zur Herstellung der Verbindungen der Formel (IV) setzt man pro Mol des 2-(2-Hydroxy-phenyl)-2-methoxyimino-acetamides der Formel (II) im Allgemeinen 1 bis 15 Mol, vorzugsweise 2 bis 8 Mol eines Trihalogenpyrimidins der allgemeinen Formel (VI) ein.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die Reaktionsdurchführung, Aufarbeitung und Isolierung der Reaktionsprodukte erfolgt nach allgemein üblichen Verfahren (vergleiche auch die Herstellungsbeispiele).

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Psdeudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, Puccinia-Arten, Fusarium-Arten und Pyrenophora-Arten, von Krankheiten im Wein-, Obst- und Gemüseanbau, wie beispielsweise gegen Venturia-, Sphaerotheca- und Plasmopara-Arten, Phytophthora-Arten oder von Reiskrankheiten, wie beispielsweise gegen Pyricularia-Arten, einsetzen. Mit gutem Erfolg werden auch weitere Getreidekrankheiten, wie beispielsweise Septoria-, Pyrenophora- oder Cochliobolus-Arten, bekämpft. Ferner lassen sich die erfindungsgemäßen Verbindungen auch zur Steigerung des Ernteertrags von Kulturpflanzen einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,

Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,

Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,

Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,

Ediphenphos, Epoxiconazol, Etaconazol, Ethirimol, Etridiazol,

Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,

Guazatin,

Hexachlorobenzol, Hexaconazol, Hymexazol,

Imazalil, Imibenconazol, Iminoctadin, Iminoctadinealbesilate, Iminoctadinetriacetate, Iodocarb, Ipconazol, Iprobenfos(IBP), Iprodione, Irumamycin, Isoprothiolan, Isovaledione,

Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,

Nickel-dimethyldithiocarbamat, Nitrothal-isopropyl, Nuarimol,

Ofurace, Oxadixyl, Oxamocarb, Oxolinicacid, Oxycarboxim, Oxyfenthiin,

Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,

Quinconazol, Quintozen(PCNB),

Schwefel und Schwefel-Zubereitungen,

Tebuconazol, Tecloftalam, Tecnazen, Tetcyclacis, Tetraconazol, Thiabendazol, Thicyofen, Thifluzamide, Thiophanate-methyl, Thiram, Tioxymid, Totclofos-methyl, Tolylfluanid, Triadimefon, Triadimenol, Triazbutil, Triazoxid, Trichlamid, Tricyclazol, Tridemorph, Triflumizol, Triforin, Triticonazol,

Uniconazol,

Validamycin A, Vinclozolin, Viniconazol,

Zarilamid, Zineb, Ziram sowie

Dagger G,

OK-8705,

OK-8801,

α-(1,1-Dimethylethyl)-β-(2-phenoxyethyl)-1H-1,2,4-triazol-1-ethanol,

α-(2,4-Dichlorphenyl)-β-fluor-β-propyl-1H-1,2,4-triazol-1-ethanol,

α-(2,4-Dichlorphenyl)-β-methoxy-α-methyl-1H-1,2,4-triazol-1-ethanol,

α-(5-Methyl-1,3-dioxan-5-yl)-β-[[4-(trifluormethyl)-phenyl]-methylen]-1H-1,2,4-triazol-1-ethanol,

(5RS,6RS)-6-Hydroxy-2,2,7,7-tetramethyl-5-(1H-1,2,4-triazol-1-yl)-3-octanon,

(E)-α-(Methoxyimino)-N-methyl-2-phenoxy-phenylacetamid,

{2-Methyl-1-[[[1-(4-methylphenyl)ethyl]amino]carbonyl]propyl}-carbaminsäure-1-isopropylester

1-(2,4-Dichlorphenyl)-2-(1H-1,2,4-triazol-1-yl)ethanon-O-(phenylmethyl)-oxim,

1-(2-Methyl-1-naphthalenyl)-1H-pyrrol-2,5-dion,

1-(3,5-Dichlorphenyl)-3-(2-propenyl)-2,5-pyrrolidindion,

1-[(Diiodmethyl)-sulfonyl]-4-methyl-benzol,

1-[[2-(2,4-Dichlorphenyt)-1,3-dioxolan-2-yt]-methyt]-1H-imidazol,

1-[[2-(4-Chlorphenyl)-3-phenyloxiranyl]-methyl]-1H-1,2,4-triazol,

1-[1-[2-[(2,4-Dichlorphenyl)methoxy]phenyl]ethenyl]-1 H-imidazol,

1-Methyl-5-nonyl-2-(phenylmethyl)-3-pyrrolidinol,

2',6'-Dibrom-2-methyl-4'-trifluormethoxy-4'-trifluor-methyl-1,3-thiazol-5-carboxanilid,

2,2-Dichlor-N-[1-(4-chlorphenyl)ethyl]-1-ethyl-3-methyl-cyclopropancarboxamid,

2,6-Dichlor-5-(methylthio)-4-pyrimidinyl-thiocyanat,

2,6-Dichlor-N-(4-trifluormethylbenzyl)-benzamid,

2,6-Dichlor-N-[[4-(trifluormethyl)phenyl]methyl]-benzamid,

2-(2,3,3- Triiod-2-propenyl)-2H-tetrazol,

2-[(1-Methylethyl)sulfonyl]-5-(trichlormethyl)-1,3,4-thiadiazol,

2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,

2-Aminobutan,

2-Brom-2-(brommethyl)-pentandinitril,

2-Chlor-N-(2, 3-dihydro-1,1,3-trimethyl-1H-inden-4-yl)-3-pyridincarboxamid,

2-Chlor-N-(2,6-dimethylphenyl)-N-(isothiocyanatomethyl)-acetamid,

2-Phenylphenol(OPP),

3,4-Dichlor-1-[4-(difluormethoxy)-phenyl]-1H-pyrrol-2,5-dion,

3,5-Dichlor-N-[cyan[(1-methyl-2-propynyl)oxy]methyl]-benzamid,

3-(1,1-Dimethylpropyl-1-oxo-1 H-inden-2-carbonitril,

3-[2-(4-Chlorphenyl)-5-ethoxy-3-isoxazolidinyl]-pyridin,

4-Chlor-2-cyan-N,N-dimethyl-5-(4-methylphenyl)-1H-imidazol-1-sulfonamid,

4-Methyl-tetrazolo[1,5-a]quinazolin-5(4H)-on,

8-(1,1-Dimethylethyl)-N-ethyl-N-propyl-1,4-dioxaspiro[4.5]decan-2-methanamin,

8-Hydroxychinolinsulfat,

9H-Xanthen-9-carbonsäure-2-[(phenylamino)-carbonyl]-hydrazid,

bis-(1-Methylethyl)-3-methyl-4-[(3-methylbenzoyl)oxy]-2,5-thiophendicarboxylat,

cis-1-(4-Chlorphenyl)-2-(1H-1,2,4-triazol-1-yl)-cycloheptanol,

cis-4-[3-[4-(1,1-Dimethylpropyl)-phenyl-2-methylpropyl]-2,6-dimethyl-morpholin-hydrochlorid,

Ethyl-[(4-chlorphenyl)-azo]-cyanoacetat,

Kaliumhydrogencarbonat,

Methantetrathiol, -Natriumsalz,

Methyl-1-(2,3-dihydro-2,2-dimethyl-1H-inden-1-yl)-1H-imidazol-5-carboxylat,

Methyl-N-(2,6-dimethylphenyl)-N-(5-isoxazolylcarbonyl)-DL-alaninat,

Methyl-N-(chloracetyl)-N-(2,6-dimethylphenyl)-DL-alaninat,

N-(2,3-Dichlor-4-hydroxyphenyl)-1-methyl-cyclohexancarboxamid.

N-(2,6-Dimethylphenyl)-2-methoxy-N-(tetrahydro-2-oxo-3-furanyl)-acetamid,

N-(2,6-Dimethylphenyl)-2-methoxy-N-(tetrahydro-2-oxo-3-thienyl)-acetamid,

N-(2-Chlor-4-nitrophenyl)-4-methyl-3-nitro-benzolsulfonamid,

N-(4-Cyclohexylphenyl)-1,4,5,6-tetrahydro-2-pyrimidinamin,

N-(4-Hexylphenyl)-1,4, 5,6-tetrahydro-2-pyrimidinamin,

N-(5-Chlor-2-methylphenyl)-2-methoxy-N-(2-oxo-3-oxazolidinyl)-acetamid,

N-(6-Methoxy)-3-pyridinyl)-cyclopropancarboxamid,

N-[2,2,2-Trichlor-1-[(chloracetyl)-amino]-ethyl]-benzamid,

N-[3-Chlor-4,5-bis(2-propinyloxy)-phenyl]-N'-methoxy-methanimidamid,

N-Formyl-N-hydroxy-DL-alanin, -Natriumsalz,

O,O-Diethyl-[2-(dipropylamino)-2-oxoethyl]-ethylphosphoramidothioat,

O-Methyl-S-phenyl-phenylpropylphosphoramidothioat,

S-Methyl-1,2,3-benzothiadiazol-7-carbothioat,

spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,

Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,

Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin,

Lamda-cyhalothrin, Lufenuron,

Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,

Naled, NC 184, Nitenpyram

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,

Quinalphos,

Salithion, Sebufos, Silafluofen, Sulfotep, Sulprofos,

Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,

Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im Allgemeinen zwischen 1 und 0,0001 Gew.-%, vorzugsweise zwischen 0,5 und 0,001 Gew.-%.

Bei der Saatgutbehandlung werden im Allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1

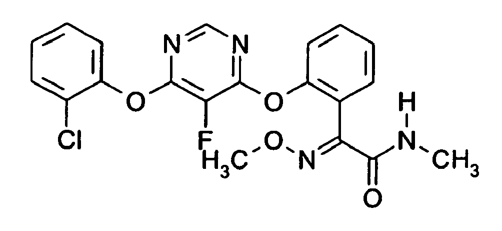

### Verfahren a)

Zu einer Mischung aus 2 g (0,0096 Mol) 2-(2-Hydroxy-phenyl)-2-methoxyimino-N-methylacetamid und 2,3 g (0,0095 Mol) 4-(2-Chlorphenoxy)-5,6-difluorpyrimidin in 10 ml Dimethylformamid gibt man unter Kühlen 0,4 g (0,01 Mol) 60 %iges Natriumhydrid zu und rührt 12 Stunden bei 25°C. Man gießt das Reaktionsgemisch auf Wasser, extrahiert mit Dichlormethan, trocknet die organische Phase über Natriumsulfat und destilliert das Lösungsmittel im Vakuum ab. Der Rückstand wird mit einer Mischung aus gleichen Volumina Essigsäureethylester und Cyclohexan auf Kieselgel chromatographiert. Man erhält 2,1 g (48,3 % der Theorie) 2-{2-[6-(2-Chlorphenoxy)-5-fluor-pyrimidin-4-yloxy]-phenyl}-2-methoxyimino-N-methyl-acetamid.

¹H-NMR-Spektrum (CDCl₃/TMS): ### = 2,88/2,90 (3H); 3,82 (3H); 6,68 (1H); 7,25-7,54 (8H); 8,05 (1H) ppm.

### Beispiel 2

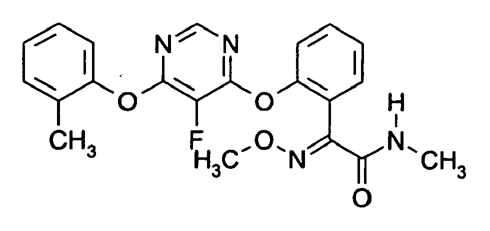

### Verfahren b)

Zu einer Mischung aus 2 g (0,0062 Mol) 2-[2-(5,6-Difluorpyrimidin-4-yloxy)-phenyl]-2-methoxyimino-N-methyl-acetamid und 0,67 g (0,0062 Mol) 2-Methylphenol in 20 ml Dimethylformamid gibt man unter Kühlen 0,25 g (0,0062 Mol) 60 %iges Natriumhydrid zu und rührt 12 Stunden bei 25°C. Man gießt das Reaktionsgemisch auf Wasser, extrahiert mit Essigsäureethylester, trocknet die organische Phase über Natriumsulfat und destilliert das Lösungsmittel im Vakuum ab. Man erhält 1,5 g (58,9 %) 2-[2-(5-Fluor-6-o-tolyloxy-pyrimidin-4-yloxy)-phenyl]-2-methoxyimino-N-methyl-acetamid.

¹H-NMR-Spektrum (CDCl₃/TMS): ### = 2,21 (3H); 2,89/2,90(3H); 3,84 (3H); 6,7 (1H, b); 7,06-7,53 (8H); 8,06 (1H) ppm.

Analog den Beispielen 1 bis 2, sowie entsprechend den Angaben in der allgemeinen Verfahrensbeschreibung, werden die in der nachstehenden Tabelle 1 genannten Verbindungen der Formel (Ia) erhalten.

**Tabelle 1:**

| | | | | | | | |
|---|---|---|---|---|---|---|---|
| 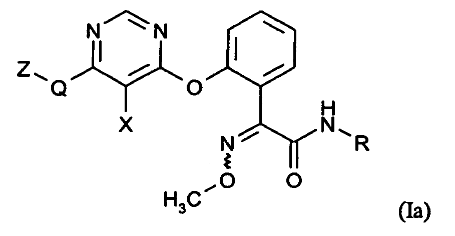 | | | | | | | |

| **Bsp.- Nr.** | **Z** | **Q** | **X** | **R** | **Fp.** (°C) | **NMR *** | **LogP* *** |
|---|---|---|---|---|---|---|---|
| **3** | Phenyl | O | F | -CH₃ | 107 | 3,85 | 2,77 |
| **4** | 2-Cyanphenyl | O | F | -CH₃ | 128- | 3,85 | 2,6 |
| | | | | | 130 | | |
| 5 | 2-Methoxyphenyl | O | F | -CH₃ | | 3,83 | 2,72 |
| 2 | 2-Methylphenyl | O | F | -CH₃ | | 3,84 | 3,04 |
| 6 | 4-Chlorphenyl | O | F | -CH₃ | | 3,85 | 3,22 |
| 7 | 2-Acetylphenyl | O | F | -CH₃ | | 3,85 | 2,51 |
| 8 | 2-Allyloxyphenyl | O | F | -CH₃ | | 3,82 | 3,11 |
| 9 | 2-Propionyloxyphenyl | O | F | -CH₃ | | 3,85 | 2,83 |
| 10 | 2-Chlorphenyl | O | F | -H | | 3,87 | 2,79 |
| 11 | 2-Bromphenyl | O | F | -CH₃ | | 3,83 | 3,08 |
| 12 | 2-Fluorphenyl | O | F | -CH₃ | | 3,82 | 2,85 |
| 13 | 2,4-Dibromphenyl | O | F | -CH₃ | | 3,83 | 3,76 |
| 14 | 2,3-Dichlorphenyl | O | F | -CH₃ | | 3,83 | 3,45 |
| 15 | 2,4-Dichlorphenyl | O | F | -CH₃ | | 3,83 | 3,61 |
| 16 | 2,5-Dichlorphenyl | O | F | -CH₃ | | 3,83 | 3,53 |
| 17 | 2,6-Dichlorphenyl | O | F | -CH₃ | | 3,79 | 3,35 |
| 18 | 2,3-Dimethylphenyl | O | F | -CH₃ | | 3,77 | 3,30 |
| 19 | 2,4-Dimethylphenyl | O | F | -CH₃ | | 3,84 | 3,39 |
| 20 | 2,5-Dimethylphenyl | O | F | -CH₃ | | 3,76 | 3,52 |
| 21 | 2,6-Dimethylphenyl | O | F | -CH₃ | | 3,82 | 3,29 |
| 22 | 2-Chlor-4-methylphenyl | O | F | -CH₃ | | 3,82 | 3,41 |
| 23 | 2-Chlor-5-methylphenyl | O | F | -CH₃ | | 3,82 | 3,37 |
| 24 | 3-Chlor-2-methylphenyl | O | F | -CH₃ | | 3,84 | 3,50 |
| 25 | 4-Chlor-2-methylphenyl | O | F | -CH₃ | | | |
| 26 | 2-Brom-4-chlorphenyl | O | F | -CH₃ | | 3,83 | 3,65 |
| 27 | 4-Brom-2-chlorphenyl | O | F | -CH₃ | | 3,82 | 3,70 |
| 28 | Phenyl | S | F | -CH₃ | | 3,81 | 3,07 |

| | | | | | | | |
|---|---|---|---|---|---|---|---|
| *) Die ¹H-NMR-Spektren wurden in Deuterochloroform (CDCl₃) oder Hexa-deuterodimethylsulfoxid (DMSO-d₆) mit Tetramethylsilan (TMS) als innerem Standard aufgenommen. Angegeben ist die chemische Verschiebung als 8-Wert in ppm. **) Die Bestimmung der logP-Werte erfolgte gemäß EEC-Directive 79/831 Annex V. A8 durch HPLC (Gradientenmethode, Acetonitril/0,1 % wässrige Phosphorsäure) | | | | | | | |

### Herstellung der Ausgangsstoffe nach Formel (II):

### Beispiel (II-1):

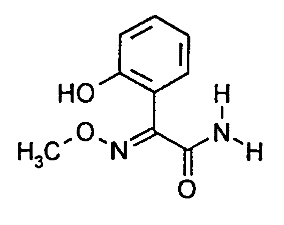

5 g (0,028 Mol) Benzofuran-2,3-dion-3-(O-methyl-oxim) (WO-A 9524396) werden in 100 ml Tetrahydrofuran mit 20 ml 25 %iger wäßriger Ammoniaklösung 2 Stunden bei 20°C gerührt. Dann destilliert man das Lösungsmittel im Vakuum ab, gießt den Rückstand auf Wasser, extrahiert mit Essigsäureethylester, trocknet die organische Phase über Natriumsulfat und destilliert das Lösungsmittel im Vakuum ab. Man kristallisiert den Rückstand aus Ethanol um und erhält 2 g (36,4 % der Theorie) 2-(2-Hydroxy-phenyl)-2-methoxyimino-acetamid.

log p = 0,83.

GC/MS silyliert:

Retentionsindex = 1827

M = 341, 323, 307, 291, 149, 133, 192, 176, 135, 116, 89, 73, 45, 26.

### Herstellung von Ausgangsstoffen nach Formel (III)

### Beispiel (III-1)

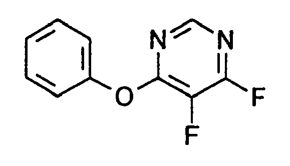

Eine Lösung von 42,4 g (0,45 Mol) Phenol und 50,4 g (0,45 Mol) Kalium-tert.-butylat in 400 ml Tetrahydrofuran tropft man bei 0°C zu einer Lösung von 80 g (0,6 Mol) 4,5,6-Trifluorpyrimidin in 11 Tetrahydrofuran. Nach vollendeter Zugabe rührt man 30 Minuten bei 0°C, gießt dann das Reaktionsgemisch auf Wasser und extrahiert mit Essigsäureethylester. Man trocknet die organische Phase über Natriumsulfat, engt im Vakuum ein und verrührt den Rückstand mit tiefsiedendem Petrolether. Man erhält 63,8 g (68,1 % der Theorie) 4-Phenoxy-5,6-difluorpyrimidin vom Schmelzpunkt 65 - 66°C.

### Herstellung der Ausgangsstoffe nach Formel (IV):

### Beispiel (IV-1)

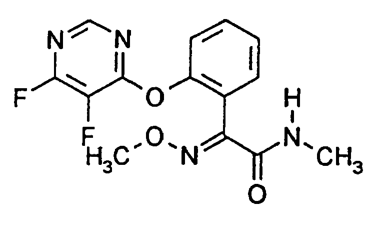

5 g (0,024 Mol) 2-(2-Hydroxy-phenyl)-2-methoxyimino-N-methyl-acetamid werden in 30 ml Tetrahydrofuran gelöst und auf 0°C gekühlt. Man gibt portionsweise unter Rühren 2,7 g (0,024 Mol) Kalium-tert.-butylat zu. Bei 0°C tropft man die so erhaltene Lösung in eine Lösung von 4,5,6-Trifluorpyrimidin in 40 ml Tetrahydrofuran. Hierauf rührt man die Mischung eine Stunde bei 20°C. Dann destilliert man das Lösungsmittel im Vakuum ab, gießt den Rückstand auf Wasser, extrahiert mit Essigsäureethylester, trocknet die organische Phase über Natriumsulfat und destilliert das Lösungsmittel im Vakuum ab. Nach Verrühren mit Diethylether filtriert man 3,2 g (41,3 % der Theorie) kristallines 2-[2-(5,6-Difluor-pyrimidin-4-yloxy)-phenyl]-2-methoxyimino-N-methylacetamid ab.

¹H-NMR-Spektrum (CDCl₃/TMS): δ = 2,87/2,88 (3H); 3,81 (3H); 6,67 (1H, b); 7,33-7,55 (4H); 8,19/8,20 (1H) ppm.

### Herstellung eines Vorproduktes nach Formel (VI)

### Beispiel (VI-1)

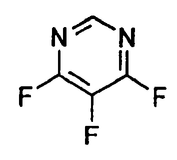

Aus einer Mischung von 609 g Kaliumfluorid in 2,3 1 Sulfolan werden zur Trocknung 500 ml Flüssigkeit bei 145°C und 20 mbar abdestilliert. Anschließend werden 1054 g 5-Chlor-4,6-difluorpyrimidin (DE-A 3843558) und 25 g Tetraphenylphosphoniumbromid zugegeben, 5 bar Stickstoff aufgedrückt und 24 Stunden bei 240°C gerührt, wobei der Druck bis 11 bar steigt. Die Reaktionsmischung wird auf 80°C gekühlt und entspannt. Nun wird die Mischung bei Normaldruck wieder langsam erhitzt, wobei das Produkt abdestilliert. Hat die Sumpftemperatur 200°C erreicht, wird der Druck auf 150 mbar vermindert, um die Destillation zu beschleunigen und um weiteres Produkt zu erhalten. Insgesamt erhält man 664 g (70,7 % der Theorie) 4,5,6-Trifluorpyrimidin vom Siedepunkt 86 bis 87°C.

### Anwendungsbeispiele

### Beispiel A:

**Plasmopara-Test** (Reben) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Plasmopara viticola* inokuliert und verbleiben dann 1 Tag in einer Inkubationskabine bei 20°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele (1), (2), (3), (12), (13), (14), (15), (16), (17), (18), (20), (22), (23), (24), (26), (27) und (28) bei einer beispielhaften Aufwandmenge an Wirkstoff von 100 g/ha einen Wirkungsgrad von 94% oder mehr im Vergleich zur unbehandelten Kontrolle.

### Beispiel B:

**Sphaerotheca-Test** (Gurke) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Sphaerotheca fuliginea* inokuliert. Die Pflanzen werden dann im Gewächshaus bei ca. 23°C und einer relativen Luftfeuchtigkeit von ca. 70 % im Gewächshaus aufgestellt..

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigt z.B. die folgenden Verbindungen der Herstellungsbeispiele (1), (2), (3), (6), (7), (9), (12), (13), (14), (15), (17), (18), (20), (21), (22), (23), (24), (26), (27) und (28) bei einer beispielhaften Aufwandmenge an Wirkstoff von 100 g/ha einen Wirkungsgrad von 91 % oder mehr im Vergleich zur unbehandelten Kontrolle.

### Beispiel C:

**Venturia-Test** (Apfel) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigt z.B. die folgenden Verbindungen der Herstellungsbeispiele (2), (3), (4), (5), (6), (8), (9), (12), (14), (17), (18), (21) und (28) bei einer beispielhaften Aufwandmenge an Wirkstoff von 10 g/ha einen Wirkungsgrad von 96 % oder mehr im Vergleich zur unbehandelten Kontrolle.

### Beispiel D:

**Erysiphe-Test** (Gerste) / protektiv

| | |
|---|---|
| Lösungsmittel: | 10 Gewichtsteile N-Methyl-pyrrolidon |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von *Erysiphe graminis f.sp. hordei* bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigt z.B. die folgenden Verbindungen der Herstellungsbeispiele (2), (3) und (8) bei einer beispielhaften Wirkstoffaufwandmenge von 250 g/ha einen Wirkungsgrad von 100 % im Vergleich zur unbehandelten Kontrolle.

### Beispiel E

Erysiphe-Test (Gerste) / kurativ

| | |
|---|---|
| Lösungsmittel: | 10 Gew.-Teile N-Methyl-pyrrolidon |
| Emulgator: | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (2), (3), (6), (9) und (10) aufgeführten erfindungsgemäßen Stoffe bei einer Aufwandmenge von 250 g/ha einen Wirkungsgrad von 90 % oder mehr.

### Beispiel F:

**Pyricularia-Test** (Reis) / protektiv

| | |
|---|---|
| Lösungsmittel: | 12,5 Gewichtsteile Aceton |
| Emulgator: | 0,3 Gewichtsteile Alkylarylpolyglykolether |

Zur Herstellung einer zweckmäßigen Wirkstoffzubereitung vermischt man I Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. 1 Tag nach dem Antrocknen des Spritzbelages werden die Pflanzen mit einer wäßrigen Sporensuspension von *Pyricularia oryzae* inokuliert. Anschließend werden die Pflanzen in einem Gewächshaus bei 100% rel. Luftfeuchtigkeit und 25°C aufgestellt.

4 Tage nach der Inokulation erfolgt die Auswertung.

Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigt z.B. die folgenden Verbindungen der Herstellungsbeispiele (1), (2), (3), (4), (8), (9), (10), (20), (21), (22) und (24) bei einer beispielhaften Aufwandmenge an Wirkstoff von 759'0 g/ha einen Wirkungsgrad von 80 % im Vergleich zur unbehandelten Kontrolle.

### Beispiel G

Puccinia-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (2), (5), (12), (13), (14), (15), (16), (24) und (26) aufgeführten erfindungsgemäßen Stoffe bei einer Aufwandmenge von 250 g/ha einen Wirkungsgrad von 90 % oder mehr.

### Beispiel H

Fusarium nivale (var. nivale)-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Fusarium nivale (var. nivale) besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (2), (13), (14), (15), (16), (17), (20), (21), (22), (24) und (26) aufgeführten erfindungsgemäßen Stoffe bei einer Aufwandmenge von 250 g/ha einen Wirkungsgrad von 90 % oder mehr.

### Beispiel I

Pyrenophora teres-Test (Gerste) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (17) und (28) aufgeführten erfindungsgemäßen Stoffe bei einer Aufwandmenge von g/ha einen Wirkungsgrad von 90 % oder mehr.

### Beispiel K

Phytophthora-Test (Tomate) / protektiv

| | |
|---|---|
| Lösungsmittel: | 47 Gewichtsteile Aceton |
| Emulgator: | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (8 (9) und (10) aufgeführten erfindungsgemäßen Stoffe bei einer Aufwandmenge von g/ha einen Wirkungsgrad von 96 % oder mehr.

## Patentansprüche

1. Verbindungen der allgemeinen Formel (I), 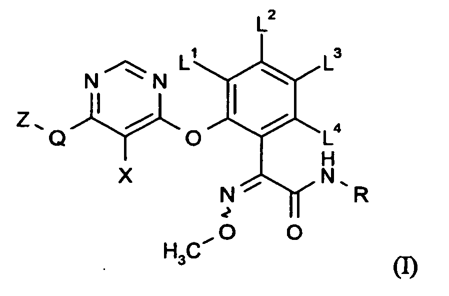 in welcher
Z für jeweils gegebenenfalls einfach bis zweifach durch Halogen, Alkyl, oder Hydroxy substituiertes Cycloalkyl mit 3 bis 7 Kohlenstoffatomen; für gegebenenfalls durch Alkyl mit 1 bis 4 Kohlenstoffatomen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder. Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis- 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 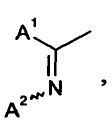 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
R für Wasserstoff oder Methyl steht,
Q für Sauerstoff oder Schwefel steht und
X für Fluor, Chlor, Brom oder Iod steht,
L¹, L², L³ und L⁴ gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen.

2. Verbindungen der Formel (I) gemäß Anspruch 1, in welcher
Z für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl oder Ethyl substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 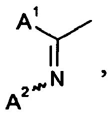 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie
jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl; Dioxol-2-yl, Oxadiazolyl,
R für Wasserstoff oder Methyl steht,
Q für Sauerstoff oder Schwefel steht und
X für Fluor oder Chlor steht und
L¹, L², L³ und L⁴ gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen.

3. Verbindungen der Formel (I) gemäß Anspruch 1 in denen Q für Sauerstoff steht.

4. Schädlingsbekämpfungsmittel, **gekennzeichnet durch** einen Gehalt an mindestens einer Verbindung der Formel (I) nach Anspruch 1.

5. Verfahren zur Bekämpfung von Schädlingen, **dadurch gekennzeichnet, dass** man Verbindungen der Formel (I) nach Anspruch 1 auf Schädlinge und/oder ihren Lebensraum einwirken lässt.

6. Verwendung von Verbindungen der Formel (I) nach den Ansprüchen 1 bis 3 zur Bekämpfung von Schädlingen.

7. Verfahren zur Herstellung von Schädlingsbekämpfungsmitteln, **dadurch gekennzeichnet, dass** man Verbindungen der Formel (I) nach den Ansprüchen 1 bis 4 mit Streckmitteln und/oder oberflächenaktiven Mitteln vermischt.

8. Verbindungen der Formel (IV) 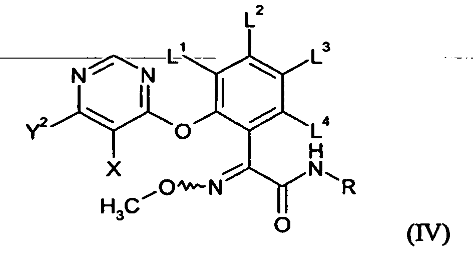 in welcher
L¹, L², L³ und L⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen,
R für Wasserstoff oder Alkyl steht,
X für Halogen steht und
Y² für Halogen steht.

9. Verfahren zur Herstellung von Verbindungen der Formel (I) 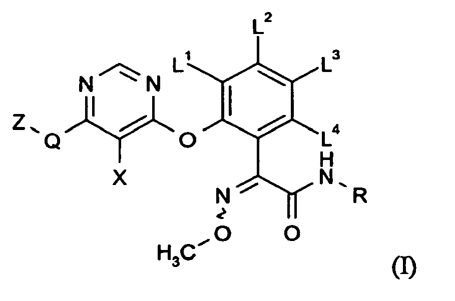 in welcher
Z für jeweils gegebenenfalls substituiertes Cycloalkyl, Aryl oder Heterocyclyl steht,
R für Wasserstoff oder Alkyl steht,
Q für Sauerstoff oder Schwefel steht und
X für Halogen steht,
L¹, L², L³ und L⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen.
**dadurch gekennzeichnet, dass** man
a) 2-(2-Hydroxy-phenyl)-2-methoxyimino-acetamide der Formel (II), 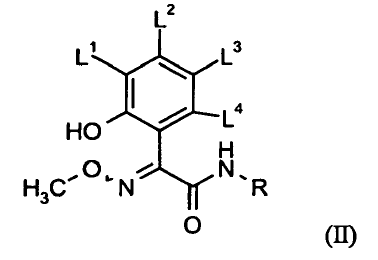 in welcher
R, L¹, L², L³ und L⁴ die oben angegebenen Bedeutungen haben,
mit einem substituierten Halogenpyrimidin der allgemeinen Formel (III), 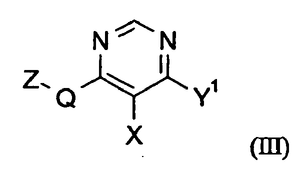 in welcher
Z, Q und X die oben angegebenen Bedeutungen haben und
Y¹ für Halogen steht,
gegebenenfalls in Gegenwart eines Verdünnungsmittels, gegebenenfalls in Gegenwart eines Säureakzeptors und gegebenenfalls in Gegenwart eines Katalysators, umsetzt, oder dass man
b) Phenoxypyrimidine der allgemeinen Formel (IV) 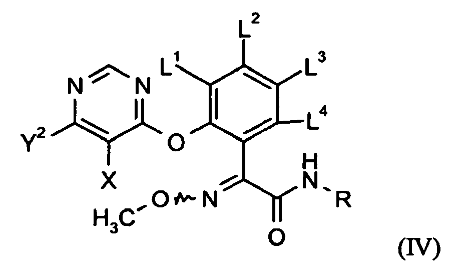
in welcher
R, L¹, L², L³, L⁴ und X die oben angegebenen Bedeutungen haben
und
Y² für Halogen steht,
mit einer Ringverbindung der allgemeinen Formel (V),
Z-Q-H (V)
in welcher
Z und Q die oben angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators, umsetzt.

10. Verbindung der Formel 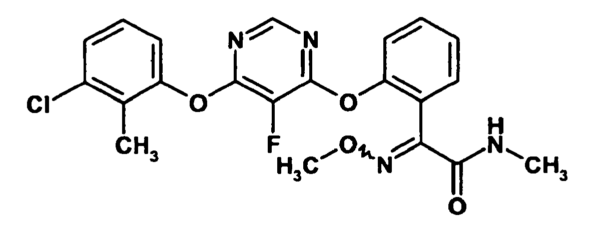 gemäß Anspruch 1.

11. Verbindung der Formel 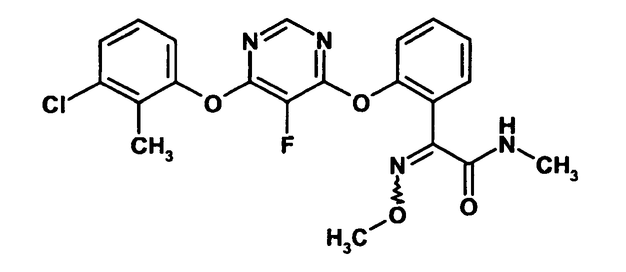 gemäß Anspruch 1.

## Claims

1. Compounds of the general formula (I) 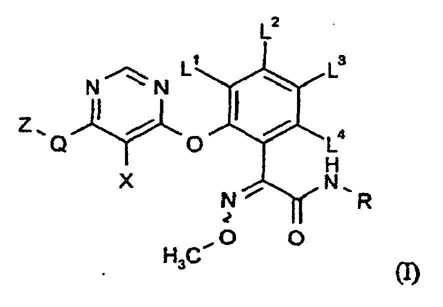 in which
Z represents cycloalkyl having 3 to 7 carbon atoms which is in each case optionally mono- to disubstituted by halogen, alkyl or hydroxyl;
represents heterocyclyl having 3 to 7 ring members which is optionally substituted by alkyl having 1 to 4 carbon atoms;
or represents phenyl or naphthyl, each of which is optionally mono- to tetrasubstituted by identical or different substituents, the possible substituents preferably being selected from the list below:
halogen, cyano, nitro, amino, hydroxyl, formyl, carboxyl, carbamoyl, thiocarbamoyl;
respectively straight-chain or branched alkyl, hydroxyalkyl, oxoalkyl, alkoxy, alkoxyalkyl, alkylthioalkyl, dialkoxyalkyl, alkylthio, alkylsulphinyl or alkylsulphonyl having in each case 1 to 8 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 11 identical or different halogen atoms;
respectively straight-chain or branched alkylamino, dialkylamino,
alkylcarbonyl, alkylcarbonyloxy, alkoxycarbonyl, alkylaminocarbonyl, dialkylaminocarbonyl, arylalkylaminocarbonyl, dialkylaminocarbonyloxy, alkenylcarbonyl or alkinylcarbonyl having 1 to 6 carbon atoms in the respective hydrocarbon chains;
cycloalkyl or cycloalkyloxy having in each case 3 to 6 carbon atoms;
alkylene having 3 or 4 carbon atoms, oxyalkylene having 2 or 3 carbon atoms or
dioxyalkylene having 1 or 2 carbon atoms, each of which is attached doubly and in each case optionally mono- to tetrasubstituted by identical or different substituents from the group consisting of fluorine, chlorine, oxo, methyl, trifluoromethyl and ethyl;
or a grouping 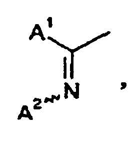 in which
A¹ represents hydrogen, hydroxyl or alkyl having 1 to 4 carbon atoms or cycloalkyl having 1 to 6 carbon atoms and
A² represents hydroxyl, amino, methylamino, phenyl, benzyl or represents respectively optionally cyano-, hydroxyl-, alkoxy-, alkylthio-, alkylamino-, dialkylamino- or phenyl-substituted alkyl or alkoxy having 1 to 4 carbon atoms, or represents alkenyloxy or alkinyloxy having in each case 2 to 4 carbon atoms,
and phenyl, phenoxy, phenyllthio, benzoyl, benzoylethenyl, cinnamoyl, heterocyclyl or phenylalkyl, phenylalkyloxy, phenylalkylthio or heterocyclylalkyl having in each case 1 to 3 carbon atoms in the respective alkyl moieties, each of which is optionally mono- to trisubstituted in the ring moiety by halogen and/or straight-chain or branched alkyl or alkoxy having 1 to 4 carbon atoms,
R represents hydrogen or methyl,
Q represents oxygen or sulphur and
X represents fluorine, chlorine, bromine or iodine,
L¹, L², L³ and L⁴ are identical or different and, independently of one another, each represents hydrogen, halogen, cyano, nitro, or represents alkyl, alkoxy, alkylthio, alkylsulphinyl or alkylsulphonyl having in each case 1 to 6 carbon atoms and being in each case optionally substituted by 1 to 5 halogen atoms.

2. Compounds of the formula (I) according to Claim 1 in which
Z represents cyclopentyl or cyclohexyl, each of which is optionally mono- to disubstituted by fluorine, chlorine, methyl, ethyl or hydroxyl;
represents optionally methyl- or ethyl-substituted thienyl, pyridyl or furyl;
or represents phenyl or naphthyl, each of which is optionally mono- to tetrasubstituted by identical or different substituents, the possible substituents being selected from the list below:
fluorine, chlorine, bromine, iodine, cyano, nitro, amino, hydroxyl, formyl, carboxyl, carbamoyl, thiocarbamoyl,
methyl, ethyl, n- or i-propyl, n-, i-, s- or t-butyl, 1-, 2-, 3-, neo-pentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimetylbutyl), 1-, 2-(2,3-dimethylbutyl),
hydroxymethyl, hydroxyethyl, 3-oxobutyl, methoxymethyl, dimethoxymethyl,
methoxy, ethoxy, n- or i-propoxy,
methylthio, ethylthio, n- or i-propylthio, methylsulphinyl, ethylsulphinyl, methylsulphonyl or ethylsulphonyl,
vinyl, allyl, 2-methylallyl, propen-1-yl, crotonyl, propargyl, vinyloxy, allyloxy, 2-methylallyloxy, propen-1-yloxy, crotonyloxy, propargyloxy;
trifluoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
methylamino, ethylamino, n- or i-propylamino, dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
propanediyl, ethyleneoxy, methylenedioxy, ethylenedioxy, each of which is doubly attached and in each case optionally mono- to
tetrasubstituted by identical or different substituents from the group consisting of fluorine, chlorine, oxo, methyl or trifluoromethyl,
or a grouping 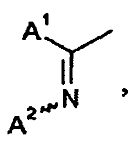 where
A¹ represents hydrogen, methyl or hydroxyl and
A² represents hydroxyl, methoxy, ethoxy, amino, methylamino, phenyl, benzyl or hydroxyethyl, and
phenyl, phenoxy, phenyllthio, benzoyl, benzoylethenyl, cinnamoyl, benzyl, phenylethyl, phenylpropyl, benzyloxy, benzylthio, 5,6-dihydro-1,4,2-dioxazin-3-ylmethyl, triazolylmethyl, benzoxazol-2-ylmethyl, 1,3-dioxan-2-yl, benzimidazol-2-yl, dioxol-2-yl, oxadiazolyl, each of which is optionally mono- to trisubstituted in the ring moiety by halogen and/or straight-chain or branched alkyl or alkoxy having 1 to 4 carbon atoms,
R represents hydrogen or methyl,
Q represents oxygen or sulphur and
X represents fluorine or chlorine and
L¹, L², L³ and L⁴ are identical or different and, independently of one another, each represents hydrogen, fluorine, chlorine, bromine, cyano, nitro, methyl, ethyl, n- or i-propyl, n-, i-,
s- or t-butyl, methoxy, ethoxy, n- or i- propoxy, methylthio, ethylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl.

3. Compounds of the formula (I) according to Claim 1 in which Q represents oxygen.

4. Pesticides, **characterized in that** they comprise at least one compound of the formula (I) according to Claim 1.

5. Method for controlling pests, **characterized in that** compounds of the formula (I) according to Claim 1 are allowed to act on pests and/or their habitat.

6. Use of compounds of the formula (I) according to Claims 1 to 3 for controlling pests.

7. Process for preparing pesticides, **characterized in that** compounds of the formula (I) according to Claims 1 to 4 are mixed with extenders and/or surface-active agents.

8. Compounds of the formula (IV) 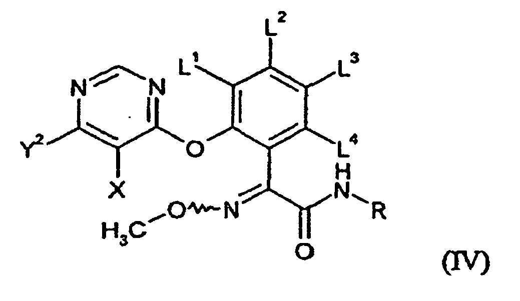 in which
L¹, L², L³ and L⁴ are identical or different and, independently of one another, each represent hydrogen, halogen, cyano, nitro, respectively optionally halogen-substituted alkyl, alkoxy, alkylthio, alkylsulphinyl or alkylsulphonyl,
R represents hydrogen or alkyl,
X represents halogen and
Y² represents halogen.

9. Process for preparing compounds of the formula (I) 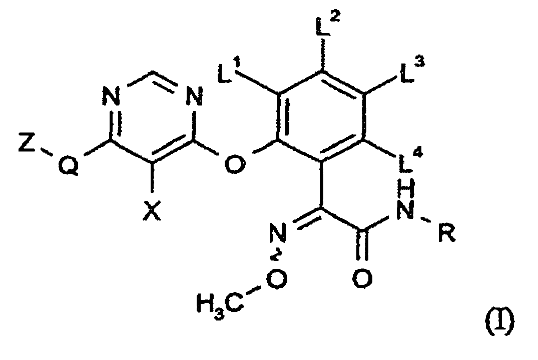 in which
Z represents respectively optionally substituted cycloalkyl, aryl or heterocyclyl,
R represents hydrogen or alkyl,
Q represents oxygen or sulphur and
X represents halogen,
L¹, L², L³ and L⁴ are identical or different and, independently of one another, each represent hydrogen, halogen, cyano, nitro, respectively optionally halogen-substituted alkyl, alkoxy, alkylthio, alkylsulphinyl or alkylsulphonyl.
**characterized in that**
a) 2-(2-hydroxyphenyl)-2-methoxyimino-acetamides of the formula (II) 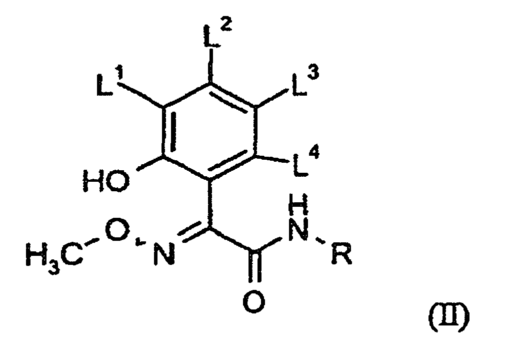 in which
R, L¹, L², L³ and L⁴ are each as defined above
are reacted with a substituted halogenopyrimidine of the general formula (III) 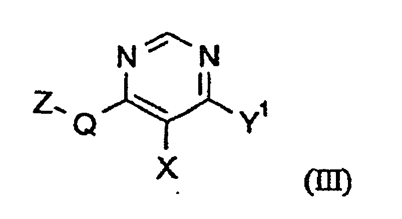 in which
Z, Q, R and X are each as defined above and
Y¹ represents halogen,
if appropriate in the presence of a diluent, if appropriate in the presence of an acid acceptor and if appropriate in the presence of a catalyst, or
b)phenoxypyrimidines of the general formula (IV) 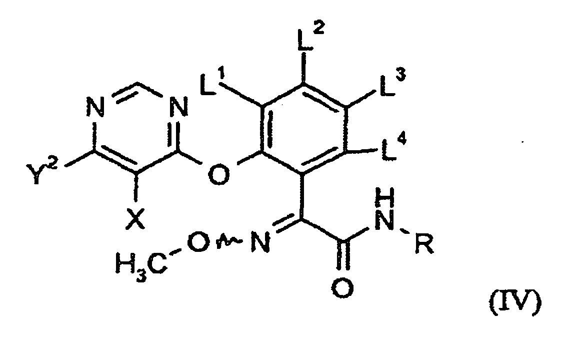 in which
R, L¹, L², L³, L⁴ and X are each as defined above and
Y² represents halogen
are reacted with a ring compound of the general formula (V)
Z-Q-H (V)
in which
Z and Q are each as defined above,
if appropriate in the presence of a diluent, if appropriate in the presence of an acid acceptor and if appropriate in the presence of a catalyst.

10. Compound of the formula 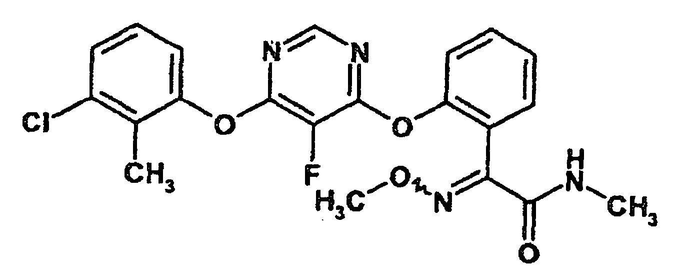 according to Claim 1.

11. Compound of the formula 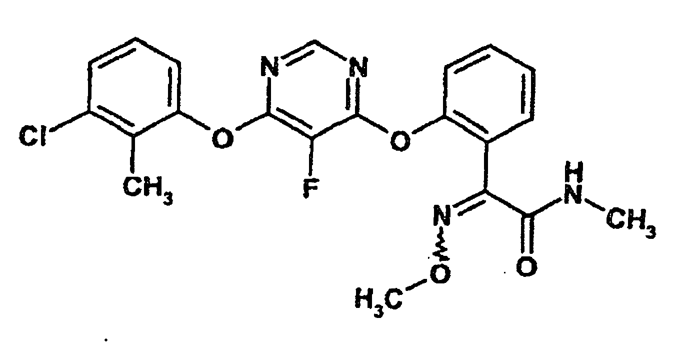 according to Claim 1.

## Revendications

1. Composés de formule générale (I), 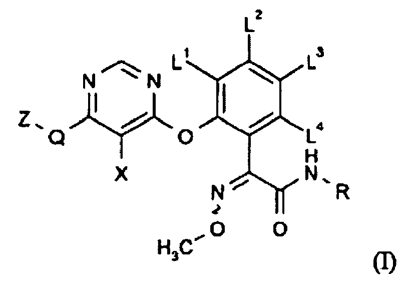 dans laquelle
Z désigne un reste cycloalkyle ayant 3 à 7 atomes de carbone, éventuellement substitué dans chaque cas une ou deux fois par un radical halogéno, alkyle ou hydroxy ; un reste hétérocyclyle trigonal à heptagonal éventuellement substitué par un radical alkyle ayant 1 à 4 atomes de carbone ;
ou bien un reste phényle ou naphtyle chacun éventuellement substitué une à quatre fois identiques ou différentes, les substituants possibles étant choisis parmi ceux qui sont énumérés ci-après :
halogéno, cyano, nitro, amino, hydroxy, formyle, carboxy, carbamoyle, thiocarbamoyle ;
un reste alkyle, hydroxyalkyle, oxoalkyle, alkoxy, alkoxyalkyle, alkylthioalkyle, dialkoxyalkyle, alkylthio, alkylsulfinyle ou alkylsulfonyle ayant chacun 1 à 8 atomes de carbone et chacun étant linéaire ou ramifié ;
un reste alcényle ou alcényloxy ayant chacun 2 à 6 atomes de carbone et chacun étant linéaire ou ramifié ;
un reste halogénalkyle, halogénalkoxy, halogénalkylthio, halogénalkylsulfinyle ou halogénalkylsulfonyle, chacun linéaire ou ramifié, ayant chacun 1 à 6 atomes de carbone et 1 à 13 atomes d'halogène identiques ou différents ;
un reste halogénalcényle ou halogénalcényloxy chacun linéaire ou ramifié ayant chacun 2 à 6 atomes de carbone et 1 à 11 atomes d'halogène identiques ou différents ; un reste alkylamino, dialkylamino chacun linéaire ou ramifié,
un reste alkylcarbonyle, alkylcarbonyloxy, alkoxycarbonyle, alkylaminocarbonyle, dialkylaminocarbonyle, arylalkylaminocarbonyle, dialkylaminocarbonyloxy, alcénylcarbonyle ou alcynylcarbonyle, ayant 1 à 6 atomes de carbone dans les chaînes hydrocarbonées respectives ;
un reste cycloalkyle ou cycloalkyloxy ayant chacun 3 à 6 atomes de carbone ;
un reste alkylène ayant 3 ou 4 atomes de carbone, oxyalkylène ayant 2 ou 3 atomes de carbone ou dioxyalkylène ayant 1 ou 2 atomes de carbone à deux liaisons dans chaque cas, chacun éventuellement substitué une à quatre fois identiques ou différentes par un radical fluoro, chloro, oxo, méthyle, trifluorométhyle ou éthyle ;
ou bien un groupement 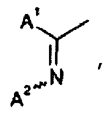 dans lequel
A¹ représente l'hydrogène, un groupe hydroxy ou un reste alkyle ayant 1 à 4 atomes de carbone ou cycloalkyle ayant 1 à 6 atomes de carbone et
A² représente un groupe hydroxy, amino, un reste méthylamino, phényle, benzyle ou un reste alkyle ou alkoxy ayant 1 à 4 atomes de carbone, chacun éventuellement substitué par un radical cyano, hydroxy, alkoxy, alkylthio, alkylamino, dialkylamino ou phényle, ou bien un reste alcynyloxy ayant chacun 2 à 4 atomes de carbone,
ainsi qu'un reste phényle, phénoxy, phénylthio, benzoyle, benzoyléthényle, cinnamoyle, hétérocyclyle ou phénylalkyle, phénylalkyloxy, phénylalkylthio ou hétérocyclylalkyle ayant chacun 1 à 3 atomes de carbone dans les parties alkyle respectives et chacun étant éventuellement substitué dans la partie cyclique une à trois fois par un radical halogéno et/ou par un radical alkyle ou alkoxy linéaire ou ramifié ayant 1 à 4 atomes de carbone,
R représente l'hydrogène ou un reste méthyle,
Q représente l'oxygène ou le soufre, et
X représente le fluoro, le chlore, le brome ou l'iode,
L¹, L², L³ et L⁴ sont identiques ou différents et représentent dans chaque cas, indépendamment les uns des autres, l'hydrogène, un halogène, un groupe cyano, nitro, un reste alkyle, alkoxy, alkylthio, alkylsulfinyle ou alkylsulfonyle ayant chacun 1 à 6 atomes de carbone et chacun étant éventuellement substitué par 1 à 5 atomes d'halogène.

2. Composés de formule (I) suivant la revendication 1, formule dans laquelle
Z désigne un reste cyclopentyle ou cyclohexyle, chacun éventuellement substitué une ou deux fois par un radical fluoro, chloro, méthyle, éthyle ou hydroxy;
un reste, éventuellement substitué par un radical méthyle ou éthyle, thiényle, pyridyle ou furyle ;
ou bien un reste phényle ou naphtyle, chacun éventuellement substitué une à quatre fois identiques ou différentes, les substituants possibles étant choisis parmi ceux qui sont énumérés ci-après :
fluor, chlore, brome, iode, cyano, nitro, amino, hydroxy, formyle, carboxy, carbamoyle, thiocarbamoyle,
méthyle, éthyle, n-propyle, isopropyle, n-butyle, isobutyle, sec.-butyle, tertiobutyle, 1-, 2-, 3-néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-, 2-(2,3-diméthylbutyle), hydroxyméthyle, hydroxyéthyle, 3-oxobutyle, méthoxyméthyle, diméthoxyméthyle, méthoxy, éthoxy, n-propoxy ou isopropoxy,
méthylthio, éthylthio, n-propylthio, isopropylthio, méthylsulfinyle, éthylsulfinyle, méthylsulfonyle ou éthylsulfonyle,
vinyle, allyle, 2-méthylallyle, propène-1-yle, crotonyle, propargyle, vinyloxy, allyloxy, 2-méthylallyloxy, propène-1-yloxy, crotonyloxy, propargyloxy ;
trifluorométhyle, trifluoréthyle,
difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle,
méthylamino, éthylamino, n-propylamino, isopropylamino, diméthylamino, diéthylamino,
acétyle, propionyle, méthoxycarbonyle, éthoxycarbonyle, méthylaminocarbonyle, éthylaminocarbonyle, diméthylaminocarbonyle, diéthylaminocarbonyle, diméthylaminocarbonyloxy, diéthylaminocarbonyloxy, benzylaminocarbonyle, acryloyle, propioloyle,
cyclopentyle, cyclohexyle,
propanediyle, éthylènoxy, méthylènedioxy, éthylènedioxy, ayant chacun deux liaisons et chacun éventuellement substitué une à quatre fois identiques ou différentes par du fluor, du chlore, un radical oxo, méthyle ou trifluorométhyle,
ou bien un groupement 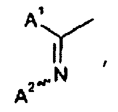 dans lequel
A¹ représente l'hydrogène, un reste méthyle ou un groupe hydroxy
A² représente un groupe hydroxy, un reste méthoxy, éthoxy, un groupe amino, un reste méthylamino, phényle, benzyle ou hydroxyéthyle, ainsi que
un reste phényle, phénoxy, phénylthio, benzoyle, benzoyléthényle, cinnamoyle, benzyle, phényléthyle, phénylpropyle, benzyloxy, benzylthio, 5,6-dihydro-1,4,2-dioxazine-3-ylméthyle, triazolylméthyle, benzoxazole-2-ylméthyle, 1,3-dioxanne-2-yle, benzimidazole-2-yle, dioxol-2-yle, oxadiazolyle, chacun éventuellement substitué dans la partie cyclique une à trois fois par un radical halogéno et/ou un radical alkyle ou alkoxy linéaire ou ramifié ayant 1 à 4 atomes de carbone,
R représente l'hydrogène ou un reste méthyle,
Q représente l'oxygène ou le soufre et
X représente le fluor ou le chlore, et
L¹, L², L³ et L⁴ sont identiques ou différents et représentent, indépendamment les uns des autres, chacun l'hydrogène, le fluor, le chlore, le brome, un groupe cyano, nitro, un reste méthyle, éthyle, n-propyle, isopropyle, n-butyle, isobutyle, sec.-butyle, tertiobutyle, méthoxy, éthoxy, n-propoxy, isopropoxy, méthylthio, éthylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle ou trifluorométhylsulfonyle.

3. Composés de formule (I) suivant la revendication 1, dans lesquels Q représente l'oxygène.

4. Compositions pesticides, **caractérisées par** une teneur en au moins un composé de formule (I) suivant la revendication 1.

5. Procédé pour combattre des parasites, **caractérisé en ce qu'**on fait agir des composés de formule (I) suivant la revendication 1 sur des parasites et/ou sur leur milieu.

6. Utilisation de composés de formule (I) suivant les revendications 1 à 3 pour combattre des parasites.

7. Procédé de préparation de compositions pesticides, **caractérisé en ce qu'**on mélange des composés de formule (I) suivant les revendications 1 à 4 avec des diluants et/ou des agents tensioactifs.

8. Composés de formule (IV) 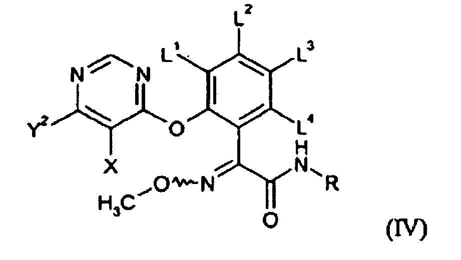 dans laquelle
L¹, L², L³, et L⁴ sont identiques ou différents et représentent, indépendamment les uns des autres, chacun l'hydrogène, un halogène, un groupe cyano, nitro, un reste alkyle, alkoxy, alkylthio, alkylsulfinyle ou alkylsulfonyle, chacun éventuellement substitué par un halogène,
R représente l'hydrogène ou un reste alkyle,
X représente un halogène et
Y² représente un halogène.

9. Procédé de production de composés de formule (I) 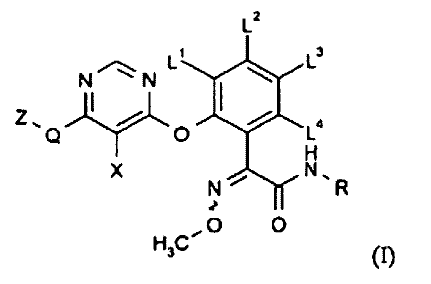 dans laquelle
Z est un reste cycloalkyle, aryle ou hétérocyclyle chacun éventuellement substitué,
R représente l'hydrogène ou un reste alkyle,
Q représente l'oxygène ou le soufre et
X représente un halogène
L¹, L ² L³ et L⁴ , sont identiques ou différents et représentent, indépendamment les uns des autres, chacun l'hydrogène, un halogène, un groupe cyano, nitro, un reste alkyle, alkoxy, alkylthio, alkylsulfinyle ou alkylsulfonyle, chacun éventuellement substitué par un halogène,
**caractérisé en que** :
a) on fait réagir des 2-(2-hydroxyphényl)-2-méthoxyimino-acétamides de formule (II), 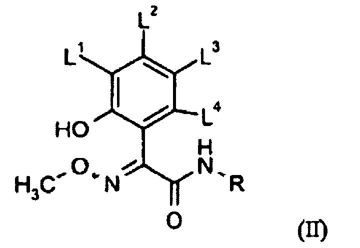 dans laquelle
R, L¹, L², L³ et L⁴ ont les définitions indiquées ci-dessus, avec une halogénopyrimidine substituée de formule générale (III) 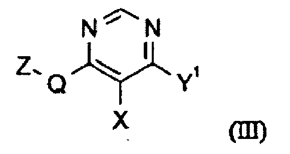 dans laquelle
Z, Q et Y ont les définitions indiquées ci-dessus, et Y¹ représente un halogène,
éventuellement en présence d'un diluant, le cas échéant en présence d'un accepteur d'acide et, éventuellement, en présence d'un catalyseur ou en ce que
b) l'on fait réagir des phénoxypyrimidines de formule générale (IV) 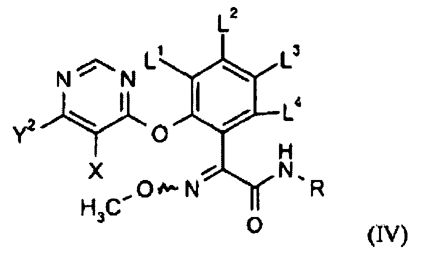
dans laquelle
R, L¹, L², L³, L⁴ et X ont les définitions indiquées ci-dessus et
Y² représente un halogène
avec un composé cyclique de formule générale (V)
Z-Q-H (V)
dans laquelle
Z et Q ont les définitions indiquées ci-dessus,
éventuellement en présence d'un diluant, le cas échéant en présence d'un accepteur d'acide et, éventuellement, en présence d'un catalyseur.

10. Composé de formule 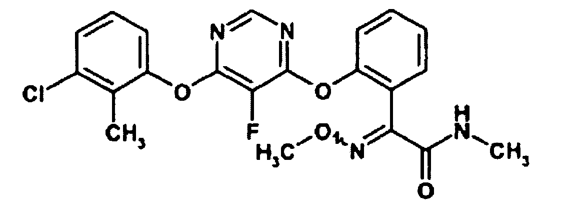 suivant la revendication 1.

11. Composé de formule 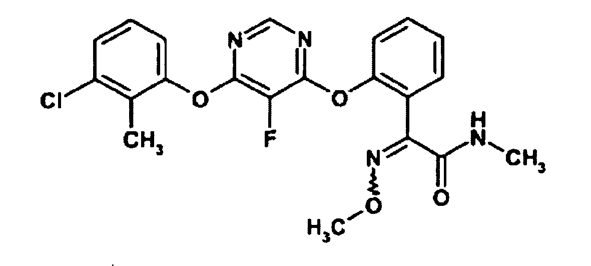 suivant la revendication 1.
